# EUROPEAN PATENT APPLICATION

(11) **EP 1 935 451 A1**
(43) Date of publication of application: **25.06.2008**
(21) Application number: 07023024.8
(22) Date of filing: 28.11.2007
(51) Int. Cl.: A61N 1/39, A61B 5/00, A61B 5/046, A61N 1/372

(54) **Implantable medical device with transmission of an atrial fibrillation message**

(30) Priority: 21.12.2006 US 614298
(71) Applicant: BIOTRONIK CRM Patent AG, 6341 Baar (CH)
(72) Inventor: Albus, Marco, Dr., 12527 Berlin (DE); Diem, Björn Henrik, Dr., 12161 Berlin (DE); Müssig, Dirk, Dr., West Linn, OR 97068 (US); Kraetschmer, Hannes, Dr., West Linn, OR 97068 (US)
(74) Representative: Lindner-Vogt, Karin L.

(57) **Abstract**

An implantable medical device has an atrial fibrillation detector adapted to detect an atrial fibrillation. A telemetry unit is adapted to transmit an AF message to an external receiver. A control unit is connected to said atrial fibrillation detector and said telemetry unit and is adapted to only indicate an atrial fibrillation detected by the atrial fibrillation detector that lasts longer than a predetermined waiting time period of at least several hours. Additionally the implantable medical device has an atrial shock generator which is connected or connectable to an atrial defibrillation electrode and adapted to generate and deliver an atrial cardioversion pulse when triggered and the control unit is connected to the atrial shock generator.

## Description

The invention refers to a medical device system for the management of the treatment of atrial fibrillation. The system includes an implant such as a cardiac pacemaker or an implantable cardioverter/defibrillator that can detect atrial fibrillation and can transmit, via a relay device, this information to a telematic application that can display this information to the physician. With this specific information from the pacemaker or the implantable cardioverter/defibrillator the physician can manage the appropiate therapy, e.g. anticoagulation or cardioversion. In addition the implantable cardioverter/defibrillator device can deliver an atrial cardioversion therapy or an atrial defibrillation shock to an atrium of a heart suffering from atrial fibrillation (AF).

Fibrillation is a particular form of tachycardia that may occur as well in an atrium (atrial fibrillation) as in a ventricle (ventricular fibrillation) of a heart. Other forms of tachycardia are for example flutter. A tachycardia is characterized by a rapid heart rate. Typically, fibrillation is characterized by a very high rate of contraction of the heart chamber (atrium or ventricle) affected and of very low amplitude of the sensed electrical potential. Typically, during an episode of fibrillation, no coordinated contraction of the whole heart chamber occurs but only a circulating excitation of the myocardium wherein only one part of the heart chamber's muscle (the myocardium) is exited (depolarised) and thus contracted, whereas other parts of the myocardium already are repolarized and thus relaxed. Therefore, during episodes of fibrillation, the affected heart chamber is unable to efficiently pump blood. For this reason, a ventricular fibrillation (VF) usually is lethal if not treated within minutes or seconds. On the other hand, an atrial fibrillation usually is not life threatening, since the atrial contraction only contributes to a smaller part to the total pumping power of the heart that is typically expressed as an minute volume: pumped blood volume per minute.

Even though atrial fibrillation or atrial flutter is not life threatening, there are several reasons for treating atrial fibrillation although such treatment is painful for the patient.

Atrial fibrillation is the most common cardiac arrhythmia. The risk of developing atrial fibrillation increases with age - AF affects four percent of individuals in their 80s. An individual may spontaneously alternate between AF and a normal rhythm (paroxysmal atrial fibrillation) or may continue with AF as the dominant cardiac rhythm without reversion to the normal rhythm (chronic atrial fibrillation). Atrial fibrillation is often asymptomatic, but may result in symptoms of palpitations, fainting, chest pain, or even heart failure. These symptoms are especially common when atrial fibrillation results in a heart rate which is either too fast or too slow. In addition, the erratic motion of the atria leads to blood stagnation (stasis) which increases the risk of blood clots that may travel from the heart to the brain and other areas. Thus, AF is an important risk factor for stroke, the most feared complication of atrial fibrillation.

Observational studies suggest that one in four to five strokes is due to atrial fibrillation. Depending on the risk profile of an individual patient, the yearly risk for a stroke is between 2% and 14%.

Also, atrial fibrillation is compromising the heart's performance because of the loss of atrioventricular synchrony associate with an atrial fibrillation and can cause discomfort as for example fatigue.

Atrial fibrillation occurs in many variants. Often it occurs just for an episode and spontanously switches back to normal sinusrhythm after some hours without any additional therapy. This is called paroxysmal atrial fibrillation. On the other hand atrial fbrillation can persist and go over into a chronic status. This is called chronic atrial fibrillation. To restore normal sinusrhythm it is nesscesary to deliver adequat therapy to the patient.

A typical treatment of a fibrillation is a cardioversion back to normal sinusrhythm or/and an anticoagulation therapy that reduces the risk of throboembolic complications.

During a cardioversion procedure an ECG-triggered electrical current is delivered via a cardioverter/defibrillator device to the patient. This resets the heart back to normal rhythm. Normally an anticoagulation therapy must be performed for about 4 weeks before a cardioversion to avoid throboembolic complications. The only exception of the procedure can be made if in the first 48h after the occurrence of atrial fibrillation a cardioversion can be performed. In this case, without providing this long-term anticoagulation, an acute cardioversion can be made.

Another issue that has to be seen is the efficiency of a cardioversion, that is reduced the longer AF is present.

The problem in daily clinical practise is to differ between the episodes of paroxysmal atrial fibrillation and the transition into chronic atrial fibrillation regarding the time window of 48h.

It is an object of the invention to provide implantable medical device that provides an improved response to atrial fibrillation.

According to the present invention the object of the invention is achieved by an implantable medical device featuring:
an atrial fibrillation detector that is adapted to detect an atrial fibrillation
a atrial cardioversion pulse generator that is connected or can be connect to an atrial defibrillation electrode and that is adapted to generate and deliver an atrial cardioversion pulse when triggered,
a telemetry unit that is adapted to transmit an AF message to an external receiver, and
a control unit, that is connected to said atrial fibrillation detector and said atrial shock generator and said telemetry unit.

The control unit is adapted to only indicate an atrial fibrillation detected by the atrial fibrillation detector that lasts longer than a predetermined waiting time period of at least several hours.

Preferably the predetermined waiting time period is a time period in the range from 10 to 14 hours.

In one preferred embodiment of the invention, the control unit is adapted to automatically induce an atrial cardioversion therapy upon indication of an atrial fibrillation.

In an alternative preferred embodiment of the invention, the control unit is adapted to send out an AF message via the telemetry unit upon indication of an atrial fibrillation. This allows a physician or any other person including a patient having the implantable medical device implanted to decide on there own whether or not an atrial cardioversion or an atrial defibrillation shall be induced. Then, cardioversion can be induced manually.

Preferably, the control unit can be switched from an automatic atrial cardioversion mode according to the second last paragraph to a manual cardioversion mode according to the last paragraph and vice versa.

In the automatic atrial cardioversion mode, the implantable medical device will allow for spontaneous natural cardioversion prior to initiating any (painful) atrial cardioversion therapy.

In the manual atrial cardioversion mode, the implantable medical device will not unnecessarily send out an AF message potentially leading to premature action of a user or physician. Thus, a disadvantage of the prior art devices is avoided, where an AF message is immediately forwarded to a central service center and the central service center in turn sends AF event messages immediately or at the next routine message to the physician. If the AF event message would be sent out always when an AF episodes is detected this results in a high number of false positive alarms because of many spontaneous terminations of these types of episodes within the first few hours.

On the other hand, if the delay is too long the risk of thromboembolic complications rises and the effort for a cardioversion is much higher. This is avoided by initiating an action - starting cardioversion therapy or sending out an AF message - after a well defined period of time of ongoing atrial fibrillation.

Further preferred features include a control unit that is adapted to trigger an atrial cardioversion therapy in synchrony with a ventricular event.

The object of the invention is further achieved by an automatic method for treating atrial fibrillation, comprising the steps of:
detecting an atrial fibrillation,
triggering a predetermined waiting time period of several hours of duration, and
indicating an ongoing atrial fibrillation only if said predetermined waiting time period times out, wherein said predetermined waiting time period is reset prior to time out of said predetermined waiting time period if said detected atrial fibrillation ceases prior to time out of said predetermined waiting time period.

It is to be appreciated that features of preferred embodiments of the invention may be combined in any useful manner thus arriving a further preferred embodiments of the invention not explicitly mentioned in this disclosure.

The above and other aspects, features and advantages of the present invention will be more apparent from the following more particular description thereof, presented in conjunction with the following drawings wherein:
- FIG. 1: shows a dual chamber pacemaker/ atrial defibrillator/cardioverter connected to leads placed in a heart.
- FIG. 2: is a block diagram of the device of figure 1.

The following description is of the best mode presently contemplated for carrying out the invention. This description is not to be taken in a limiting sense, but is made merely for the purpose of describing the general principles of the invention. The scope of the invention should be determined with reference to the claims.

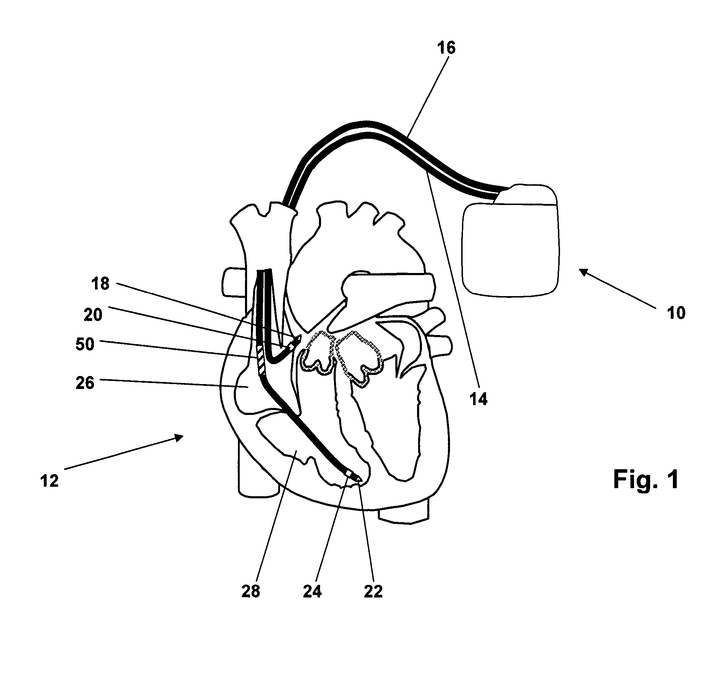

In FIG. 1 a dual chamber pacemaker 10 as heart stimulator connected to pacing/sensing leads placed in a heart 12 is illustrated. The pacemaker 10 is electrically coupled to heart 12 by way of leads 14 and 16. Lead 14 has a pair of right atrial electrodes 18 and 20 that are in contact with the right atria 26 of the heart 12. Lead 16 has a pair of electrodes 22 and 24 that are in contact with the right ventricle 28 of heart 12 and an atrial cardioversion shock coil 50 placed in atrium 32 of heart 12. Electrodes 18 and 22 are tip-electrodes at the very distal end of leads 14 and 16, respectively. Electrode 18 is a right atrial tip electrode RA-Tip and electrode 22 is a right ventricular tip electrode 22. Electrodes 20 and 24 are ring electrodes in close proximity but electrically isolated from the respective tip electrodes 18 and 22. Electrode 20 forms a right atrial ring electrode RA-Ring and electrode 24 forms a right ventricular ring electrode RV-Ring. Atrial cardioversion shock coil 50 is coil electrode providing a relatively large geometric area when compared to the stimulation electrodes 18, 20, 22 and 24.

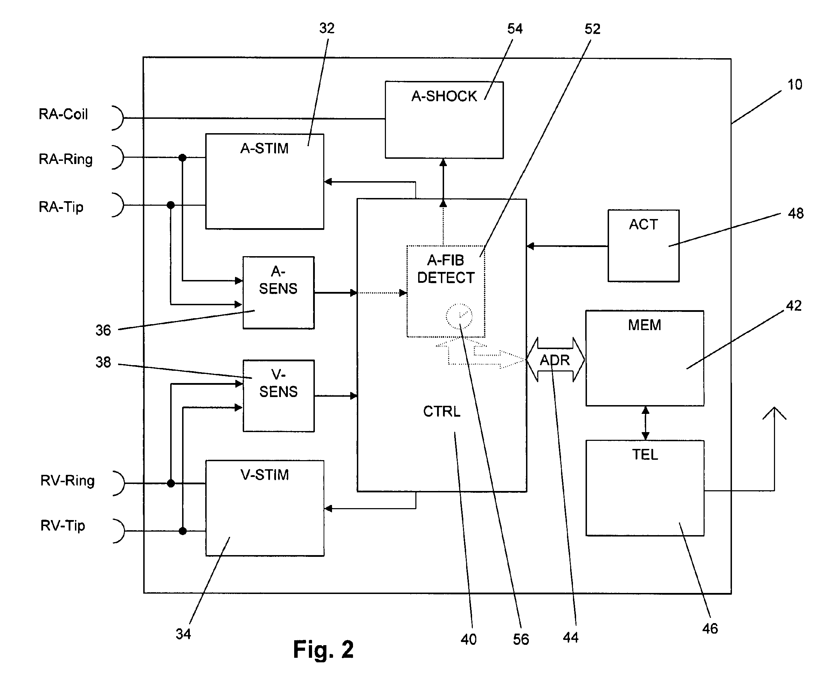

Referring to FIG. 2 a simplified block diagram of a dual chamber pacemaker 10 is illustrated. During operation of the pacemaker leads 14 and 16 are connected to respective output/input terminals of pacemaker 10 as indicated in Fig. 1 and carry stimulating pulses to the tip electrodes 18 and 22 from an atrial stimulation pulse generator A-STIM 32 and a ventricular pulse generator V-STIM 34, respectively. Further, electrical signals from the atrium are carried from the electrode pair 18 and 20, through the lead 14, to the input terminal of an atrial channel sensing stage A-SENS 36; and electrical signals from the ventricles are carried from the electrode pair 22 and 24, through the lead 16, to the input terminal of a ventricular sensing stage V-SENS 38.

Controlling the dual chamber pacer 10 is a control unit CTRL 40 that is connected to sensing stages A-SENS 36 and V-SENS 38 and to stimulation pulse generators A-STIM 32 and V-STIM 34. Control unit CTRL 40 receives the output signals from the atrial sensing stage A-SENS 36 and from the ventricular sensing stage V-SENS 38. The output signals of sensing stages A-SENS 36 and V-SENS 38 are generated each time that a P-wave representing an intrinsic atrial event or an R-wave representing an intrinsic ventricular event, respectively, is sensed within the heart 12. An As-signal is generated, when the atrial sensing stage A-SENS 36 detects a P-wave and a Vs-signal is generated, when the ventricular sensing stage V-SENS 38 detects an R-wave.

Control unit CTRL 40 also generates trigger signals that are sent to the atrial stimulation pulse generator A-STIM 32 and the ventricular stimulation pulse generator V-STIM 34, respectively. These trigger signals are generated each time that a stimulation pulse is to be generated by the respective pulse generator A-STIM 32 or V-STIM 34. The atrial trigger signal is referred to simply as the "A-pulse", and the ventricular trigger signal is referred to as the "V-pulse". During the time that either an atrial stimulation pulse or ventricular stimulation pulse is being delivered to the heart, the corresponding sensing stage, A-SENS 36 and/or V-SENS 38, is typically disabled by way of a blanking signal presented to these amplifiers from the control unit CTRL 40, respectively. This blanking action prevents the sensing stages A-SENS 36 and V-SENS 38 from becoming saturated from the relatively large stimulation pulses that are present at their input terminals during this time. This blanking action also helps prevent residual electrical signals present in the muscle tissue as a result of the pacer stimulation from being interpreted as P-waves or R-waves.

Furthermore, atrial sense events As recorded shortly after delivery of a ventricular stimulation pulses during a preset time interval called post ventricular atrial refractory period (PVARP) are generally recorded as atrial refractory sense event Aᵣₛ but ignored.

Control unit CTRL 40 comprises circuitry for timing ventricular and/or atrial stimulation pulses according to an adequate stimulation rate that can be adapted to a patient's hemodynamic need as pointed out below.

Still referring to FIG. 2, the pacer 10 includes a memory circuit MEM 42 that is coupled to the control unit CTRL 40 over a suitable data/address bus ADR 44. This memory circuit MEM 42 allows certain control parameters, used by the control unit CTRL 40 in controlling the operation of the pacemaker 10, to be programmable stored and modified, as required, in order to customize the pacemaker's operation to suit the needs of a particular patient. Such data includes the basic timing intervals used during operation of the pacemaker 10 and AV delay values and hysteresis AV delay values in particular.

Further, data sensed during the operation of the pacemaker may be stored in the memory MEM 42 for later retrieval and analysis. This includes atrioventricular interval data that are acquired by the control unit CTRL 40. Control unit CTRL 40 is adapted to determine the atrioventricular interval data as required for automatic atrioventricular interval analysis by determining the time interval between an atrial event, either sensed (As) or stimulated (Ap) and an immediately following ventricular sensed event Vs as indicated by the ventricular sensing stage V-SENS 38.

A telemetry circuit TEL 46 is further included in the pacemaker 10. This telemetry circuit TEL 46 is connected to the control unit CTRL 40 by way of a suitable command/data bus. Telemetry circuit TEL 46 allows for wireless data exchange between the pacemaker 10 and some remote programming or analyzing device, which can be part of a centralized service center serving multiple pacemakers.

The pacemaker 10 in FIG. 1 is referred to as a dual chamber pacemaker because it interfaces with both the right atrium 26 and the right ventricle 28 of the heart 12. Those portions of the pacemaker 10 that interface with the right atrium, e.g., the lead 14, the P-wave sensing stage A-SENS 36, the atrial stimulation pulse generator A-STIM 32 and corresponding portions of the control unit CTRL 40, are commonly referred to as the atrial channel. Similarly, those portions of the pacemaker 10 that interface with the right ventricle 28, e.g., the lead 16, the R-wave sensing stage V-SENS 38, the ventricular stimulation pulse generator V-STIM 34, and corresponding portions of the control unit CTRL 40, are commonly referred to as the ventricular channel.

In order to allow rate adaptive pacing in a DDDR or a DDIR mode, the pacemaker 10 further includes a physiological sensor ACT 48 that is connected to the control unit CTRL 40 of the pacemaker 10. While this sensor ACT 48 is illustrated in FIG. 2 as being included within the pacemaker 10, it is to be understood that the sensor may also be external to the pacemaker 10, yet still be implanted within or carried by the patient. A common type of sensor is an activity sensor, such as a piezoelectric crystal, mounted to the case of the pacemaker. Other types of physiologic sensors are also known, such as sensors that sense the oxygen content of blood, respiration rate, pH of blood, body motion, and the like. The type of sensor used is not critical to the present invention. Any sensor capable of sensing some physiological parameter relatable to the rate at which the heart should be beating can be used. Such sensors are commonly used with "rate-responsive" pacemakers in order to adjust the rate of the pacemaker in a manner that tracks the physiological needs of the patient.

With respect to atrial fibrillation detection and therapy, the pacemaker 10 comprises an atrial fibrillation detector 52 that is part of control unit CTRL 40 and that is connected to atrial sensing stage A-SENS 36. Further an atrial cardioversion shock generator 54 is provided that is connected via a terminal A-COIL to the atrial coil electrode 50 on ventricular electrode lead 16. The atrial cardioversion shock generator 54 is also connected to the control unit CTRL 40 and can be triggered by control unit CTRL 40 to generate an atrial cardioversion pulse or an atrial defibrillation shock when needed. Such atrial cardioversion pulse or shock for treatment of an atrial fibrillation is applied to the atrium the atrial coil electrode 50.

A defibrillation shock usually has a much higher intensity than for example a stimulation or pacing pulse. The intensity of a defibrillation shock shall be sufficient to render the whole myocardium of the fibrillating heart chamber refractory in order to interrupt a circulating excitation of the myocardium and thus to synchronize the contraction of the myocardium of the heart chamber in all it's parts.

When an atrial defibrillation shock is delivered at the wrong point of time during a heart cycle the atrial defibrillation itself can cause a ventricular fibrillation. Therefore, delivery of the atrial defibrillation shock during the so-called vulnerable phase of the ventricle is to be avoided. For this reason, control unit 40 is adapted to trigger an atrial defibrillation shock synchronously with a ventricular event. Such ventricular event may be the sensed event sensed by the ventricular sensing stage 38 or a stimulated ventricular event caused by a ventricular stimulation pulse delivered by ventricular stimulation pulse generator 34.

The control unit CTRL 40 is adapted to respond to a detection of an atrial fibrillation by means of the atrial fibrillation detector 52 by starting a timer 56 that is reset, if the atrial fibrillation should naturally cease. When not reset, the timer 56 times out after 12 hours. Then, control unit CTRL 40 either triggers the atrial cardioversion shock generator 54 or it triggers the telemetry circuit TEL 46 to send out an AF message that eventually is received by a central service center and then is forwarded to a physician or to a patient pacemaker 10 is implanted to.

Whether or not control unit CTRL 40 automatically triggers the atrial cardioversion shock generator 54 to generate and deliver an atrial cardioversion pulse or only triggers sending of an AF message depends on whether pacemaker 10 is in its automatic or in its manual AF mode. The pacemaker 10 can be switched from an automatic atrial cardioversion mode according to the second last paragraph to a manual cardioversion mode according to the last paragraph and vice versa.

Although an exemplary embodiment of the present invention has been shown and described, it should be apparent to those of ordinary skill that a number of changes and modifications to the invention may be made without departing from the spirit and scope of the invention. In particular, it is possible to implement the features of the claimed atrial fibrillation treatment system into state of the art implantable medical devices such as implantable pacemakers or implantable cardioverter/defibrillator. This invention can readily be adapted to such devices by following the present teachings. All such changes, modifications and alterations should therefore be recognized as falling within the scope of the present invention.

## Claims

1. An implantable medical device having
an atrial fibrillation detector that is adapted to detect an atrial fibrillation
a telemetry unit that is adapted to transmit an AF message to an external receiver, and
a control unit, that is connected to said atrial fibrillation detector and said telemetry unit and that is adapted to only indicate an atrial fibrillation detected by the atrial fibrillation detector that lasts longer than a predetermined waiting time period of at least several hours.

2. An implantable medical device having
an atrial fibrillation detector that is adapted to detect an atrial fibrillation
an atrial shock generator that is connected or can be connected to an atrial defibrillation electrode and that is adapted to generate and deliver an atrial cardioversion pulse when triggered,
a telemetry unit that is adapted to transmit an AF message to an external receiver, and
a control unit, that is connected to said atrial fibrillation detector and said atrial shock generator and said telemetry unit and that is adapted to only indicate an atrial fibrillation detected by the atrial fibrillation detector that lasts longer than a predetermined waiting time period of at least several hours.

3. The implantable medical device according to claim 1 or 2, wherein the predetermined waiting time period is 8 to 14 hours.

4. The implantable medical device according to one of the preceding claims 1 to 3, wherein the control unit comprises or is connected to a timer that is started when an atrial fibrillation is detected and that is either reset if an atrial fibrillation ceases prior to time out of the timer or that times out after said predetermined waiting time period, wherein the control unit is adapted to respond to a time-out of said timer by indicating an atrial fibrillation.

5. The implantable medical device according to one of the preceding claims 1 to 4, wherein the control unit is adapted to send out an AF message via the telemetry unit upon indication of an atrial fibrillation.

6. The implantable medical device according to one of the preceding claims 1 to 5, wherein the implantable medical device comprises an atrial sensing stage connected or being connectable to an electrode for picking up electric potentials inside at least an atrium of a heart, said atrial sensing stage being further connected to said atrial fibrillation detector to enable said atrial fibrillation detector to detect an atrial fibrillation by evaluating the rate atrial excitations as sensed by the atrial sensing stage.

7. The implantable medical device according to claim 2, wherein the control unit is adapted to automatically trigger said atrial shock generator upon indication of an atrial fibrillation to thus induce an atrial cardioversion therapy.

8. The implantable medical device according to claim 2, wherein the control unit can be switched from an automatic atrial cardioversion wherein the control unit automatically triggers said atrial shock generator upon indication of an atrial fibrillation to a manual cardioversion mode wherein the control unit only triggers said telemetry unit to send out an AF message.

9. The implantable medical device according to claim 2, wherein the control unit is adapted to respond to manual atrial cardioversion trigger signal received via said telemetry unit and to trigger the atrial shock generator upon reception of said manual atrial cardioversion trigger signal.

10. The implantable medical device according to claim 2, wherein the implantable medical device comprises a ventricle sensing stage connected or being connectable to an electrode for picking up electric potentials inside a ventricle of a heart and being adapted to detect intrinsic ventricular events, wherein the control unit is adapted to trigger said atrial shock generator in synchrony with a ventricular event.

11. An automatic method for treating atrial fibrillation, said method comprises the steps of:
detecting an atrial fibrillation,
triggering a predetermined waiting time period of several hours of duration, and
indicating an ongoing atrial fibrillation only if said predetermined waiting time period times out, wherein said predetermined waiting time period is reset prior to time out of said predetermined waiting time period if said detected atrial fibrillation ceases prior to time out of said predetermined waiting time period.

12. The method according to claim 11 wherein an AF message is send out if said predetermined waiting time period times out.

13. The method according to claim 11 or 12 wherein said predetermined waiting time period is between 8 and 14 hours.
